# NEW EUROPEAN PATENT SPECIFICATION

(11) **EP 1 960 005 B2**
(45) Date of publication and mention of the opposition decision: **05.11.2014**
(45) Mention of the grant of the patent: 27.07.2011
(21) Application number: 05813407.3
(22) Date of filing: 09.12.2005
(51) Int. Cl.: A61L 15/46, A61F 13/84

(54) **ABSORBENT PRODUCT**
SAUGFÄHIGES PRODUKT
PRODUIT ABSORBANT

(43) Date of publication of application: 27.08.2008
(73) Proprietor: SCA Hygiene Products AB, 405 03 Göteborg (SE)
(72) Inventor: FORSGREN BRUSK, Ulla, S-735 43 Pixbo (SE); STRIDFELDT, Chatrine, S-436 58 Hovås (SE); WALLSTRÖM, Leif, S-416 78 Göteborg (SE)
(74) Representative: Zacco Sweden AB
(86) International application number: PCT/SE2005/001883
(87) International publication number: WO 2007/067111

(56) References cited:
- EP-A- 0 510 619
- EP-A- 0 811 389
- EP-A1- 0 509 409
- EP-A1- 0 850 616
- EP-A1- 1 149 597
- EP-A1- 1 149 597
- EP-A2- 0 348 978
- US-A- 3 939 838
- US-A- 5 037 412
- US-A- 5 368 909
- US-A- 5 591 146
- US-A- 5 733 272
- US-A1- 2003 023 213
- US-B1- 6 245 693
- US-B1- 6 245 693
- US-B1- 6 417 424
- US-H- H1 732

## Description

### Technical field

The invention refers to an absorbent product, such as a diaper, a sanitary napkin or an incontinence product, having a longitudinal and a lateral direction, comprising a back sheet, being distal from the body of the wearer in use of the garment, and a top sheet, being proximal to the body of the wearer in use of the product, said product having a front part, a rear part and a crotch part lying between the front and rear parts, the product further comprising an absorbent structure, between the top and back sheet, extending longitudinally from the front part to the rear part, and whereby the product optionally comprises standing gathers, belt, elastics and/or wings, and whereby the product comprises odour control agent(s).

### Background of the invention

Prevention of odour is an extremely important comfort factor for consumers of e.g. incontinence and/or feminine products. Various kinds of odours easily arise when bodily fluids that are given off from the body of the wearer are stored in an absorbent product.

For prevention of odours one is normally focused on either (1) preventing odours from arising, or (2) preventing odours from escaping out of the absorbent product to the surrounding environment. Several different kinds of odour control agents are known for these purposes.

For instance, for preventing odours from arising a bacteria inhibitor can be used, such as cupper acetate, quaternary ammonium salts, SAP with silver and/or acidic SAP. For adsorption of odour substances, zeolites, activated carbon and/or cyclodextrin can be used. Some of these tend however to be moisture sensitive. For neutralization of acidic and/or basic odour-forming compounds, substances like baking soda, citric acid and/or acidic SAP can be used. Accordingly, different kinds of odour control agents are effective against different kinds of odour substances, and act with different mechanisms.

As an example EP-A-811389 discloses an absorbent article comprising an odour control system that can be chosen from e.g. silica, zeolite, absorbent gelling material, activated carbon, cyclodextrin and mixtures thereof. The odour control system may be layered on the absorbent core or be mixed within the core. Further, it can be distributed on the edges of the absorbent article.

Also, another problem that is associated with the odour control of absorbent products is that is difficult to prevent odours from escaping when the wearer of the product moves. This is due to the fact that a so called "pump-effect" is created when the wearer moves, which basically means that the air that is trapped between the product and the body of the wearer is pressed or pumped to the edges of the product during movement, so that it eventually escapes from the product. Hereby, odours easily escape from the product.

EP-A-347746 discloses a sanitary napkin comprising a bicomponent laminate, wherein the inner lamina has odour control properties, e.g. by inclusion of activated carbon. The lamina may be provided in a tube form positioned e.g. at the front and rear edges of the article, in order to absorb malodorous gases from passing out. One object of the first lamina is to prevent the second lamina from getting wet.

A further problem that is connected with odour inhibition is that most odours will arise in the moist environment of the absorbent structure of the absorbent product, or in another place of the product where liquid is entrapped and absorbed. Many odour-inhibiting substances are however moisture-sensitive, which means that they have a reduced effectiveness in a wet environment.

US-A-6229062 discloses an odour-controlling superabsorbent polymer having an odour-controlling compound, such as a cyclodextrin, an amphoteric surfactant, a water-insoluble phosphate, triclosan, and mixtures thereof, distributed therein. The odour-controlling SAP can be incorporated in an absorbent article.

US-A-6245693 discloses a laminated composite absorbent structure comprising a first odour absorber that is distributed in a layer beneath an absorbent gelling material and a second odour absorber that e.g. is coated on the underside of the top sheet. The first odour absorber is active when not wetted and the second odour absorber is active when wetted.

US-A-5714445 discloses an absorbent article comprising cyclodextrin and optionally another odour control agent, such as zeolites, activated carbon etc, for odour control. Cyclodextrin is preferably positioned in a position of the article where it will come in contact with liquid, in order to be most effective, such as in the core or in the fluid-receiving top sheet. Cyclodextrin can however be quite expensive to manufacture. Also, since it has a cyclic structure, its capacity to bind malodorous compounds is limited to compounds that fit into the cyclic structure and therefore the size interval of compounds that can be adsorbed by cyclodextrin is relatively small.

An additional obstacle when preventing odours from escaping from an absorbent product is that the odours, since they are in a mainly gaseous form, tend to escape from the product quickly. Thus, it is important that an odour prevention system acts efficiently, and, if possible, has the capacity to slow down the gaseous flow out of the product.

Accordingly, in order to provide an absorbent product having good odour control properties, thereby reducing the risk for undesired escaping of unpleasant odours, and for limiting odours from arising, several technical problems need to be solved.

It is the object of the present invention to solve these problems and thereby providing an absorbent product for e.g. feminine or incontinence use, which has the capacity to act both (1) where the odours originate, and (2) to prevent odours from escaping from the product after they have been formed.

### Summary of the invention

This object is achieved by providing an absorbent article in accordance with claim 1, having a wet zone, a dry zone and an interfacing zone. Hereby, means for odour control are adjusted to (1) the place of odour origin (the wet zone and possibly the interfacing zone) and (2) the places of the product where odours most easily escape (the dry zone and the interfacing zone). A new concept for dealing with odour control is thus created, combining the strengths of previously presented solutions.

Since the wet zone often will be wet during use, it is beneficial to use moisture-insensitive odour control agents in the wet zone, so that the effect of the odour control agent is not reduced. Also, it is beneficial to use odour control agents in the wet zone that have the capacity to prevent odours from arising. The dry zone, on the other hand, can e.g. be equipped with odour control agents that are moisture-sensitive, since these parts of the product normally will not be wet. The interfacing zone can be wet and/or dry, depending on the conditions during use, and thus this zone can comprise both moisture-insensitive and moisture-sensitive odour control agents, so that the odour control capacity of the entire product is optimized.

A basis for the definition of a wet zone, a dry zone and an interfacing zone is that an inner "atmosphere" will be created in an absorbent product. The air of the inner atmosphere will comprise gaseous odours that will tend to escape from the product the easiest way out, i.e. where it will be easiest for the gaseous odours to diffuse or where they due to any "pump-effect" will be pressed out of the product. By preventing odours from arising to an as large extent as possible in those parts of the product that tend to become wet (the wet zone and possibly the interfacing zone), and by entrapping created gaseous odours where they tend to escape from the product (the interfacing zone and the dry zone), the effectiveness of the odour control will be improved compared to conventional odour control solutions.

### Brief description of the drawings

Figure 1 discloses pantyliners in accordance with the invention, wherein the wet zones, the dry zones and the interfacing zones are marked.
Figure 2 discloses a diaper in accordance with the invention, wherein the wet zone, the dry zone and the interfacing zone are marked.
Figure 3 discloses a pantyliner in accordance with the invention wherein the wet zone, the dry zone and the interfacing zone are marked.
Figure 4 discloses a sanitary napkin in accordance with the invention wherein the wet zone, the dry zone and the interfacing zone are marked. The absorbent structure of this product has parts that are included mainly for stabilising purposes, and they will normally not become wet during use. Therefore they are parts of the interfacing zone.
Figure 5 discloses an absorbent product according to the invention which is equipped with a belt. The wet zone, the dry zone and the interfacing zone are marked.
Figure 6 discloses a person wearing underpants and a pantyliner showing how air that is present between the user and the pantyliner can be pressed out of the product during use.
Figure 7 discloses an absorbent product according to the invention, wherein a groove is formed in the absorbent structure.
Figure 8 is a principal view of the different layers of an absorbent product in accordance with the invention and the dry zone, the wet zone and the interfacing zone thereof.

### Detailed description of the invention

The present invention refers to an absorbent product, such as a diaper, a sanitary napkin or an incontinence product, having a longitudinal and a lateral direction, comprising a back sheet, being distal from the body of the wearer in use of the product, and a top sheet, being proximal to the body of the wearer in use of the product, said product having a front part, a rear part and a crotch part lying between the front and rear parts, the product further comprising an absorbent structure, between the top and back sheet, expending longitudinally from the front part to the rear part, and whereby the product optionally comprises standing gathers, belt, elastics and/or wings, and whereby the product comprises odour control agent(s), whereby the product has (a) a wet zone, which is intended to store liquid during use, which zone is defined by the absorbent structure, (b) a dry zone, which is intended to be substantially dry during use, which zone is defmed by the outer edges, belt, elastics, backing sheet, wings and optionally outer part of the standing gathers, and (c) an interfacing zone adjoining the wet zone, which is defined by the part(s) of the top sheet receiving liquid, optionally the inner part of the standing gathers, optionally an acquisition layer, and other parts adjoining the wet zone thereby occasionally being wet, whereby at least one substantially moisture-insensitive odour control agent is present in the wet zone and at least one odour control agent is present in the dry zone.

By "the outer edges" is meant non-absorbing parts of the product that are positioned at the outer borders of the product.

By "the wings" are meant parts of the product that are positioned along the longitudinal edges of the absorbent core, and which extend laterally, in order to e.g. secure the product to an undergarment.

Depending on the type of absorbent product in question, the borders of the wet zone, the dry zone, and the interfacing zone will vary in size and extension. Therefore the distribution of moisture-sensitive and moisture-insensitive odour control agents will vary depending on the product type.

The absorbent product can be any absorbent product in which odour control is important for the use and comfort of the wearer, such as diapers, sanitary napkins, pantiliners, incontinence garments and the like.

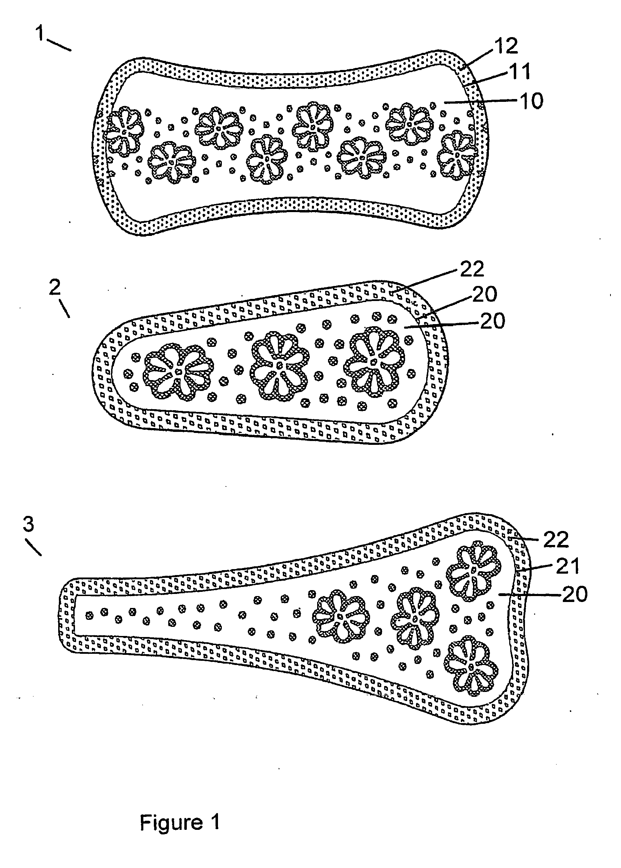

Figure 1-4 disclose different products of the invention. Figure 1 shows various pantyliners (1, 2, 3). In these products, the wet zone (10, 20, 30) is the part being capable to absorb liquid, i.e. the absorbent structure, which can be distributed to the sealing edges of the product. The interfacing zone (11, 21, 31) will be the part at the borders of the absorbent structure, i.e. basically the sealing edges and the top sheet. The dry zone (12, 22, 32) will be the parts not coming in contact with liquid, i.e. the outer part of the backing sheet and the outer edges.

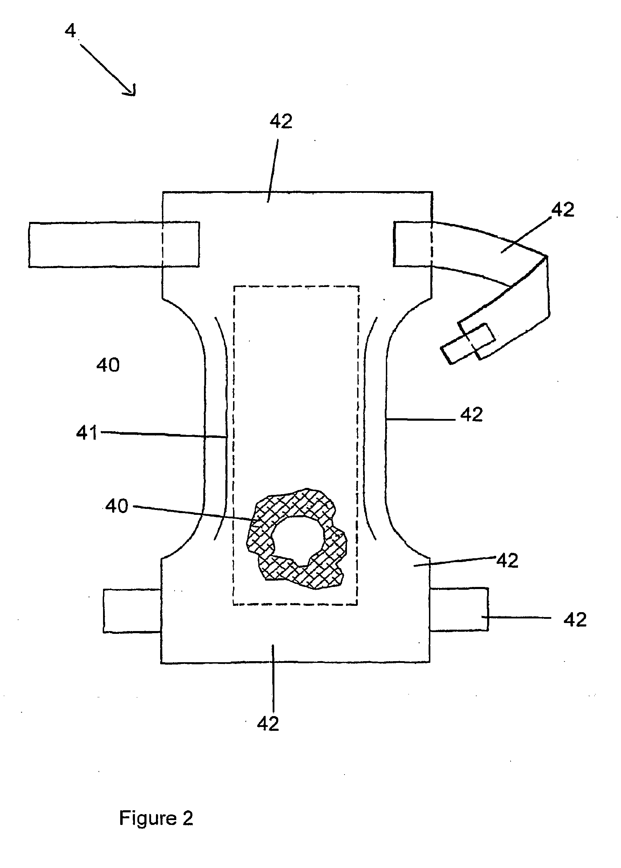

Figure 2 shows a diaper (4) in accordance with the invention. Reference numeral 40 refers to the wet zone. The interfacing zone (41) will be constituted by the parts surrounding the absorbent structure, comprising the inside of the backing sheet being in contact with the absorbent structure, the parts of the top sheet receiving liquid, and the lateral and longitudinal borders of the absorbent structure. Reference numeral 42 including the parts of the top sheet not receiving liquid refers to the dry zone.

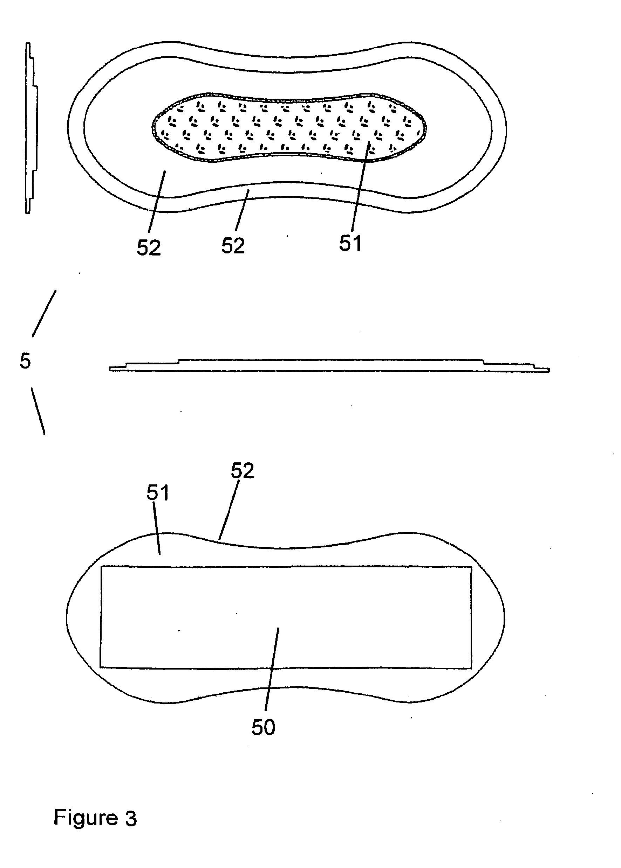

Figure 3 shows a pantyliner (5) in accordance with the invention. The liquid inlet zone of the top sheet is part of the interfacing zone (51), and other parts of the top sheet are parts of the dry zone (52). The same principles apply as for figure 1 and 2 regarding the wet zone (50).

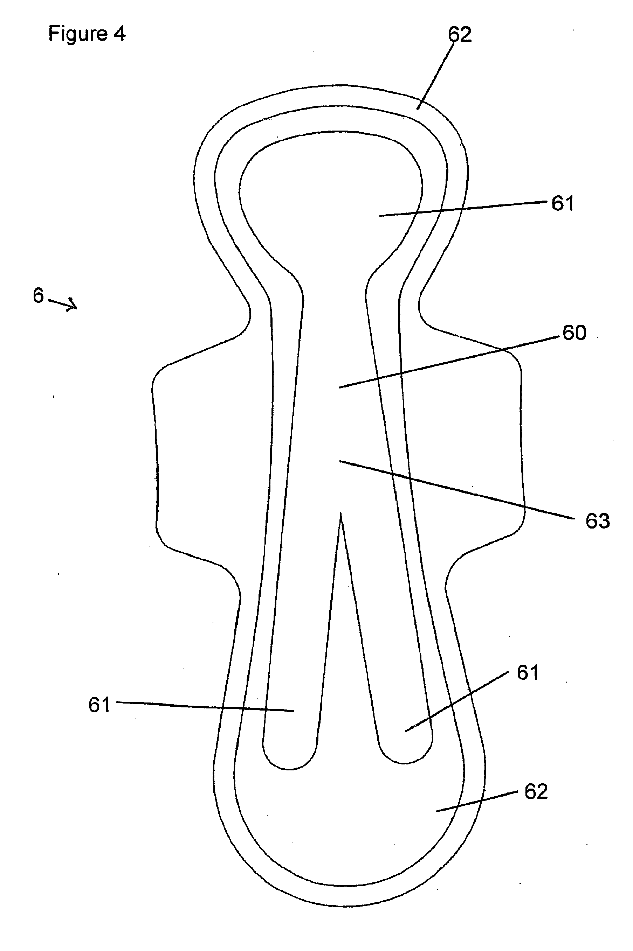

In figure 4 a sanitary napkin (6) of the present invention is shown, which napkin has an absorbent structure that is equipped with stabilising parts in the form of "legs" and "heads", i.e. the front part and the back part of the absorbent structure will primarily not store liquid but instead provide stabilising properties to the product. Hence, these parts will normally not become wetted, and thus moisture-sensitive odour control agents can be positioned in these parts of the absorbent structure, i.e. they will be parts of the interfacing zone (61). The wet zone (60), the dry zone (62) and the preliminary wet point (63) (i.e. where liquid inlet most probably will occur) are also marked.

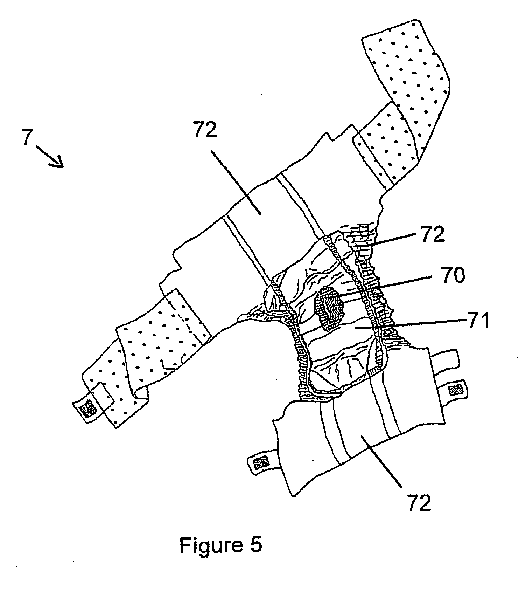

In figure 5 an absorbent product equipped with a belt (7) is shown. The same principles apply for this product as for other products of the invention with regard to the wet zone (70), the interfacing zone (71) and the dry zone (72).

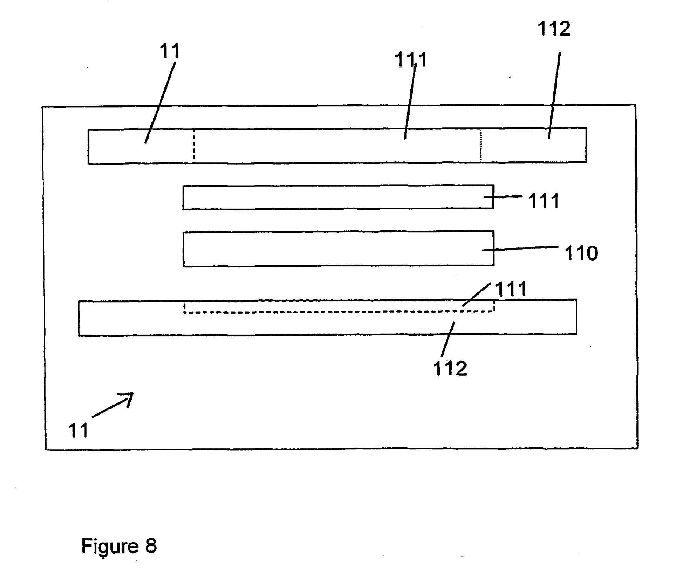

In figure 8 a principal drawing of the various layers of an absorbent product of the invention is shown. The top sheet has a liquid inlet zone where the liquid from the user is received and transported into the product. The liquid inlet zone is part of the interfacing zone (111) since it will be wet when liquid is received and drier when the liquid has been absorbed by the absorbent structure. The outer parts of the top sheet will not receive liquid and thus be part of the dry zone (112). Optionally an acquisition layer or a wadding is positioned under the top sheet. This layer will normally be part of the interfacing zone (111) since it will normally not store liquid but distribute it to the absorbent core. In products where this layer has liquid storing properties and/or purposes, it can be part of the wet zone. The absorbent structure laying under the top sheet and under the optional acquisition/wadding layer will have the function of storing liquid and hence normally the entire absorbent structure will be part of the wet zone (110). If parts of the absorbent structure have other functions, such as stabilising functions, these parts can be parts of the interfacing zone (see e.g. figure 4). The part of the backing sheet adjoining the absorbent structure will normally be parts of the interfacing zone. Other parts of the backing sheet will normally be parts of the dry zone.

Odours that arise from bacteria are primarily inhibited by preventing the growth and activity of the bacteria. Bacterial growth mainly occurs in the core, or in the areas where liquid is present, i.e. the wet zone of the present invention. Thus, in order to prevent bacterial growth it is most efficient to act in the liquid absorbing part of the absorbent product. The bacterial inhibitors can be of a type that impedes the metabolism of the bacteria or that changes the pH. It is preferable to use a low pH, which can be achieved by using an acidic SAP or an acidified pulp. Also, various metal salts, chitosan etc. can advantageously be used as odour control agents in the liquid absorbing parts of the product.

Odours that are present in the wet zone may also have other sources than bacterial origin. For example, a chemical or physical change of the absorbed liquid, or moisture in itself may give rise to odours. Odours already present will need another handling than odours having bacterial origin. In this case the role of the odour control agent is primarily to adsorb the malodorous compound(s) on a large surface or neutralise. In this case substances that have already been formed are captured by the odour control agent. Further, esters are preferably used as odour control agents, especially cyclic esters. Esters can be chosen from isomenthyl acetate, isomenthyl propionate, isomenthyl isobutyrate, isomenthyl crotonate, isomenthyl butyrate. See EP-A-1239890 for further examples of esters, Also, starch-based odour control agents, preferably having a helical configuration such as amylose, especially V-amylose, or linear dextrin, can be used in the wet zone. Especially, the starch-based odour control agent can be modified by physical or physico-chemical modification in order to achieve an enhanced specific area (preferably higher than 5 m²/g) The starch-based helical odour control agents are effective with regard to trapping hydrophobic substances.

For the dry zone may odour control agents that are blocked by e.g. liquid be suitable for use since they will act in a primarily dry environment, and thus be applied to the essentially dry parts of the product (the dry zone and possibly the interfacing zone), and not in the liquid-absorbing parts (the wet zone and possibly the interfacing zone) of the product. For example, zeolites, activated starch, polysaccharides, silica, activated carbon etc. can be used (see below for further examples).

The wet zone is defined primarily by the absorbent structure, i.e. the part of the product where liquid will be stored during use. In order to achieve an effective odour control, it is important that the odour control agents that are placed in these parts have the capacity to prevent odours from arising and to prevent odours from spreading to the outer odour control zone of the product.

The odour control agents of the wet zone comprise bacteria inhibitors and moisture-insensitive odour inhibitors. The bacterial growth inhibitor can include one or more inhibitors, and can be chosen, but are not limited to, the group comprising: chlorhexidine, quaternary ammonium compounds, cupric salts, silver and silver salts (alternatively metals or metal salts), chelating agents, parabens (methyl, ethyl and propyl), chitin, pH-buffered materials, disodium ethylenediaminetetraacetic acid (EDTA) and other chelating agents. The bacterial growth inhibitor can also include chlorhexidine digluconate. Further, the bacterial growth inhibitor can be chosen from antimicrobial agents, such as amorolfin, antibiotics, bacitracin, benzalkonium chloride, benzetonium chloride, cetrimide, fusidic acid, gentian violet (methylrosaniline chloride), hexachlorophene, hexylresorcinol, imidazole derivatives (for example biphonazole, econazole, ketoconazole, chlotrimazole, miconazole), chlorhexidine, nystatin, povidone-iodine, terbinafin, triclosan and hydrogen peroxide. Also, halogenated phenylene compounds and lactic acid bacteria can be used as well as acidic SAP/pulp and SAP with silver or other useful metals.

Moisture insensitive odour inhibitors can include one or more inhibitors, and can be chosen from, but are not limited to, the group comprising: organic or inorganic acids such as adipic acid, ascorbic acid, benzoic acid, stearic acid, boric acid, citric acid, lactic acid, tartaric acid, maleic acid polymers, malonic acid, maleic acid, polyacrylic acid and monopotassium phosphate. Also, buffers made from said acids with corresponding salts can be used. Basic compounds such as inorganic salts of carbonates, bicarbonate, phosphate, biphosphate, sulphate, bisulfate, and mixtures thereof, as those described in US-A-5037412 or as the combination of boric acid and sodium tetraborate, as disclosed in WO94/25077 , can be added. Also, Bacillus sporogenus can be included as well as polysaccharides such as chitosan, alginates, esters, cyclodextrin, silica gel, clays, diatomaceous earth, polystyrene derivates, polymeric resin materials, ion exchange resins, peat moss. Esters are preferably cyclic esters or esters chosen from isomenthyl acetate, isomenthyl propionate, isomenthyl isobutyrate, isomenthyl crotonate, isomenthyl butyrate. Also, some metal compounds and particles of metals can function as odour inhibitors.

Generally, the various odour inhibitors that are listed above will act efficiently independently of the way they are applied to the product.

The odour control agents of the wet zone should be positioned and spread evenly over the part of the product where the dominant part of the liquid of the product is present, i.e. in the absorbent core. However, this does not exclude that they are positioned in the interfacing zone (see below for further description), such as on the top sheet, in the top sheet, under the top sheet material and/or in a layer that is positioned between the top sheet and the absorbent core, such as a wadding, or at the inside of a top sheet material, such as the side of a top sheet plastic film that faces the absorbent core.

Regarding concentrations of the used odour control agents the following applies: It depends on the type of agents. The odour inhibitors of the various zones should be chosen (together) in such concentrations so that malodours arising from the article become reduced to levels undetectable by the human olfactory sense for a period of time of at least two, four or more preferably eight hours. The concentration of odour control agent will also depend on the product that is produced, i.e. products that will absorb more liquid, e.g. heavy incontinence products will be more heavily bacterially loaded than light incontinence products, and will thus need more amount of odour control agent(s). However, a skilled person in the art would know how to adjust the concentrations for the respective purpose, taking product type, odour control agent type etc into account.

For the wet zone, the following concentration intervals are well suited. With regard to e.g. acidified pulp the weight concentration of acid/buffer compared to the amount of pulp is 1- 20 %wt, preferably 1-10 %wt, and more preferably 1-5 %wt. The amount of acidic SAP in the pulp (weight % of the absorbent core) is 5-80 %wt, and preferably 10-50 %wt. SAP comprising silver or silver salts (or other metal salts): 5-50 ppm and preferably 10-30 ppm metal/metal salt in the SAP. The amount of SAP will be chosen from the absorption need and the product type, and will be easily determined by a skilled person in the art.

The method of application of the odour control agent will depend on the odour control agent. In one embodiment SAP/pulp is impregnated with the odour control agent by e.g. spraying or dipping/immensing. In another embodiment fibres that are treated with odour control agent is mixed in the absorbent core. In a further embodiment, the odour control agent can be mixed as a powder with other components of the product, such as with pulp and/or SAP. In yet another embodiment a carrier is impregnated with odour control agent (such as a bacterial inhibitor), which thereafter is positioned in, on or under the absorbent core. For example, the carrier can be of tissue material, which will be dissolved/disintegrated when it comes in contact with liquid so that the absorption capacity of the product is not impaired. The carrier can be in the form of a layer covering a part of or substantially all of the top or bottom area of the absorbent core, or it can be positioned within the absorbent core. Also, the carrier can be in the form of one or more strips that are positioned on top of or beneath or within the absorbent core. Still further, a fiber that is coated with the odour control agent can also be used. For instance, natural fibres such as bamboo fibres can act as bacterial inhibitors.

The bacterial inhibitors will interfere with the bacterial metabolism in order to avoid or to reduce the production of malodorous metabolites from the body fluids. For inhibition of bacteria, EDTA, copper acetate, SAP with silver and/or acidic SAP can e.g. be used (see the listing above). Hydrophobic malodorous compounds (such as indol, aldehydes, mercaptanes etc.) will be adsorbed by hydrophobic odour control agents and alkaline compounds such as ammonia, amines and pyridine will be neutralised by acids/acidic SAP. Thus, if several of these odour control agents are used simultaneously, a broad spectrum of malodorous compounds can be controlled.

In a preferred embodiment, more than one odour control agent is used in the wet zone and the interfacing zone, in order to provide a broad odour control spectrum, i.e. so that several different kinds of odours can be controlled. For example, in a preferred embodiment, acidic SAP is used in combination with odour control agents such as esters and/or chitosan. Hereby, neutralisation of alkaline compounds, binding of hydrophobic malodorous compounds and inhibition of bacteria is achieved simultaneously. For incontinence products, normally having to absorb much urine, bacterial inhibiting properties are normally most required for the odour control agent of the wet zone. For products absorbing blood, odour control agents based on ions (e.g. Cu, Ag) are one example. Sometimes odours may have been produced already in the urinary bladder, due to e.g. a high bacteria-content in the urine. In these cases it is beneficial if the product has the capacity to take care of the odours quickly, preferably already in the top sheet or in the wadding/acquisition layer (depending on the product type); i.e. in the interfacing layer. E.g. an acidic SAP/acidic wadding/acidic pulp are efficient for inhibiting bacterial growth. In one preferred embodiment an acidic wadding can be positioned above an acidic SAP, that is positioned within the product.

The interfacing zone is the parts of the product that can be both wet and dry during use, or will not be substantially dry during use or not substantially wet during use; the parts of the product that would not be defined as either dry or wet zone. The interfacing zone will adjoin the wet zone and can thus be wet or moisty if the wet zone is wet at the parts adjoining the interfacing zone. Also, parts of the product that are wet during liquid transport, but that may dry up since they do not have any liquid storing function, such as parts of the top sheet where liquid is taken up, and acquisition layers, wadding and inner parts of standing gathers (if standing gathers are included in the product in question), are included in the interfacing zone. Further, parts of the absorbent core that are primarily intended to stabilise the absorbent product (see e.g. figure 4) can also form part of the interfacing zone. The requirements on the interfacing zone are special, since it can be both wet and dry. Therefore, moisture-sensitive odour control agents that are positioned in the interfacing zone may lose their function and are preferably complemented with moisture-insensitive odour control agents. However, since gaseous odours may escape from the wet zone via the interfacing zone out of the product, it is beneficial if the interfacing zone have the capacity to adsorb or absorb gaseous odours. Hence, moisture-insensitive odour control agents may not be sufficient. Also, some liquid may be stored in the interfacing zone, if for instance the wet zone is completely or substantially filled with liquid, and thus it is beneficial if the odour control agent(s) of the interfacing zone has the capacity to prevent odours from arising, such as inhibiting bacterial growth.

The dry zone is defined primarily by the parts of the product that normally will not come in contact with liquid, but where gaseous odours will be spread from the wet zone before trying to escape from the product. Thus, the dry zone will comprise parts such as outer edges, belt, fastening means, elastics, backing sheet, wings, outer parts of the standing gathers and/or side panels (for diapers). By the outer edges are meant the longitudinal and lateral edges of the product in the rear, front and crotch parts of the product that constitute the "outer borders" of the product. Normally, the dry zone will be kept dry, so moisture sensitive odour control agents can be used. Also, moisture-insensitive odour control agents may of course be used. The main object of the dry zone is to prevent gaseous odours from escaping from the product, and thus the odour control agents of the dry zone should primarily be directed at entrapping gaseous odours. Preferably, the odour control agent(s) of the dry zone is(are) quick, efficient, and have a high capacity to trap gaseous odours. As a result of these requirements it is beneficial if the odour control agent of the dry zone has a high specific surface area with open positions. E.g. modified starch-based odour control agents, such as V-amylose (having a specific surface area in the order of 50-150 g/m²), activated starch (having an enhanced specific surface area of about 100-200 g/m²), linear dextrin having an enhanced specific area or activated carbon or zeolites (natural or synthetic) are suitable for use in the dry zone.

Odour control agents that can be used in the dry zone include, but are not limited to, natural zeolites, synthetic zeolites (such as for example aluminosilicate molecular sieve powders), polysaccharides, silica, activated carbon, modified starches, such as amylose, especially V-amylose, activated starch, linear dextrin or cyclodextrin, activated alumina, chlorophyll particles, hydrophobic compounds, such as paraffine, olive oil, vaseline and wax. An advantage by placing hydrophobic compounds in the dry zone is that they will not reduce/impair the absorbent capacity of the product (compared to placing them in the absorbent core). Also, hydrophobic compounds of the type suggested may have a skin caring effect. Further, the dry zone may comprise so called positive scents (i.e. contributing to a comfortable scent, such as perfumes or the like) that can be applied in an encapsulated form. The release can be controlled by temperature, moisture or mechanic action, such as friction. For amounts or concentrations of odour control agents in the dry zone, the same general principles apply as for the wet zone (see above).

Regarding application of the odour control agent in the dry zone the following principles applies. The odour control agent should be applied within or on the material(s) of the dry zone. For example, the odour control agent can be in the form of fibres having odour controlling properties. Further, the odour control agent can be sprinkled on a glue-coated surface. Also, the odour control agent can be placed in the standing gathers or in the waist elastics (for instance by clamping it between two layers in a laminate). In case the odour control agent is positioned in the waist elastics or leg elastics, it could be applied to a separate strip, or be glued to a nonwoven material that is folded to form a pocket, or it can be put in a foam structure, in the elastic thread, be coated on the fibres or on the backing sheet. Also, the odour control agent can be zoned to the parts where the malodours are likely to be transported out from the absorbent product.

The concentration that is applied for an evenly spread odour control agent, where it e.g. is glued to the backing sheet or on the material that is positioned closest to the skin, or alternatively on the wadding: 1-100 g/m², preferably 1-50 g/m², more preferably 1-30 g/m². The amount of odour control agent will vary depending on e.g. the type of agent and its capacity.

The odour control agent can be positioned in the beard or in the belt on a belt product, or in the standing gathers, so that the concentration is 1-200 g/m², preferably 1-50 g/m².

In another embodiment the odour control agent is zoned in specifically exposed areas where the concentration of odour control agent may be as high as 2500 g/m². For instance a small bag or other similar container could comprise the odour control agent, which bag could be positioned in the back part of the product in order to tighten this part (where the groove between the buttocks ends at the lower part of the back). In this case, a very high concentration of odour control agent could be necessary. Also, the odour control agent could be zoned in some parts of the product, such as in the upper front part or in the upper back edge. In these areas the concentration can be 1-200 g/m², and preferably 1-100 g/m².

In case the product is a very tight fitting product type, the malodours will normally tend to get out of the product through the breathable backing sheet. In order to reduce malodours in this case the backing sheet is coated completely or partly with an odour control agent in accordance with the invention in a concentration of e.g. 1-100 g/m², and preferably 1-50 g/m².

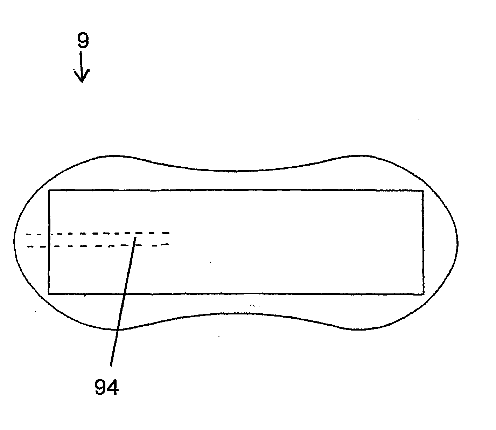

In case the odour control agent is mixed within a fibre e.g. 0.01-5.0 wt % (based on the fibre material) is used. When a fibre is coated or impregnated with an odour control agent e.g. 1-60 wt %, preferably 1-30 wt % is used (based on the fibre material). Also, grooves (94) or channels could be made in the absorbent core in order to lead the air to positions in the dry zone where the odour control agents have been concentrated (see figure 7). Hereby, a more effective odour inhibition will be the result. Also, it is important to slow down the "pumpeffect air" so that the odour control agent(s) can take care of it. Thus, the channels/grooves should have primarily two effects: (1) to temporarily store odour containing air so that it can be inhibited by an odour control agent, and (2) to direct odour containing air to one or more desired positions where an odour control agent is positioned; e.g. through a foam-based material comprising at least one odour control agent. The foam-based material can also have the effect of slowing down the air-flow out of the product. Further, by creating a space between the body of the wearer and the product (in the form of one or more grooves or channels) the "pump-effect" can be reduced since the air-pressure can be reduced in the channels/grooves compared to the comparable positions in a product lacking channels/grooves of this type. In a preferred embodiment the odour control agents can be positioned on the inside of the crotch and in the centre of the front and back of the waist part (this applies primarily for incontinence products). For feminine products the odour control agents are preferably positioned in the beard or in the wings.

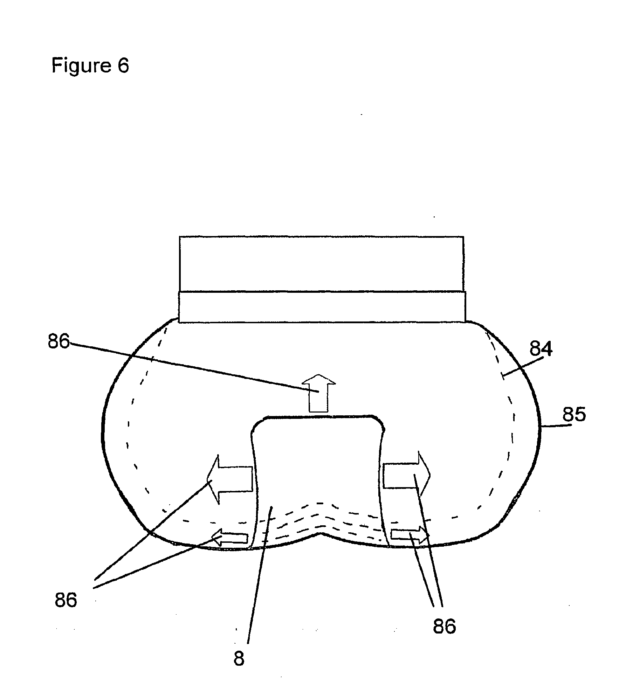

The "pump-effect" is illustrated in figure 6. Here, the buttocks of a person (84) wearing underpants (85) and a panty-liner (8) are shown. As can be seen in the figure air is present between the pantyliner and the wearer. In case the pantyliner has received liquid, the malodours may have originated from the liquid. When the wearer moves or changes position by e.g. sitting down on a chair, air is pressed out of the product (see arrows 86)). Hence it is important that odour control agents are positioned where air is pressed out of the product, so that malodours are not transported out of the product, thereby giving rise to discomfort for the wearer.

The odour control agent(s) of the dry zone can be applied in the form of a strip that is positioned so that gaseous odour that is pressed out of the product easily is trapped by the odour control agent. For example strips can e.g. be positioned along the longitudinal edges of the product in the crotch area, or in the wings of the product (preferably at the parts of the wings that are close to the absorbent core), or along the lateral edges in the rear or front part of the product.

Also, the odour control agent(s) of the dry zone can be applied to the fibres of one or more specific parts of the product. For instance, these parts can be chosen from the wings, belt, elastics, outer longitudinal or lateral edges or the backing sheet. The odour control agent(s) can be applied by spraying or coating to the fibres, or by soaking the fibres in a solution of the respective odour control agent, or in any way that will incorporate the odour control agent to the fibre. In case the odour control agent is in a powder/particle form, the agent can be applied to the material by binding or coating. For instance, if the odour control agent is applied to a plastic film material, it can be applied directly after the film has been formed (so that the film is yet not completely dry) by sprinkling the odour control agent to the film surface. Hereby, the odour control agent means will adhere to the surface of the plastic film. Also, the odour control agent(s) of the dry zone can be applied to practically the entire dry zone.

Further, the odour control agent(s) of the dry zone can be applied in spots that are positioned so that gaseous odour that is pressed out of the product easily is trapped by the odour control agent. These spots can e.g. be positioned along the longitudinal edges of the product in the crotch area, or in the wings of the product, or at the lateral edges in the rear or front part of the product. If more than one spot is applied, the distance between should be chosen so that a sufficient odour control effect is achieved; i.e. this would normally require that the spots are positioned very close to each other. The nature and concentration of the odour control agent that is used as well as the demands on the product will be parameters influencing the suitable distance; the skilled person will be capable to find suitable distances.

Moreover, if more than one odour control agent is applied to the dry zone, these can be applied in the same positions or in different positions and by different application means, as long as each odour control agent is applied in a way so that a sufficient odour control effect is achieved (see above).

Thus, in a preferred embodiment the odour control agent(s) of the dry zone is/are applied (a) as strips, (b) by being incorporated in the fibres of at least a part of the outer zone, (c) as one or more spots, (d) by being coated to the surface of the material, or a combination thereof.

In a preferred embodiment, the wet zone comprises the odour control agents acidic SAP in combination with esters and/or chitosan, the dry zone comprises modified starches and/or hydrophobic compounds, and the interfacing zone comprises acidic pulp and/or modified starches, preferably V-amylose (V=Verkleisterung).

The liquid-permeable top sheet is preferably made of a material showing properties like dryness and softness at use of the absorbent product, as this sheet lies against the body of the wearer. It is desired, that the sheet has a soft and textile-like surface, which remains dry also at repeated wettings. The top sheet may for example be composed of nonwoven material with a soft and smooth surface, such as for example a spunbond made of polypropylene fibres. In order to keep the surface closest to the skin of the wearer dry, a hydrophobic nonwoven-material may be used, which has holes, so that openings are formed in the material, which openings are greater than the cavities between the fibres of the material. In this way, fluid may be lead down through the holed openings in the top sheet to the underlying absorption core. Other examples of material in the top sheet may for example be holed plastic films, such as for example a holed polyethylene film. The top sheet may be connected to the underlying backing sheet and to the absorption core by, for example, glue or through some kind of thermal bonding.

The liquid-impermeable backing sheet consists of a flexible material, preferably a thin plastic film of PE (polyethylene), PP (polypropylene), a polyester, or some other kind of suitable material, such as a hydrophobic nonwoven-layer or a laminate of a thin film and a nonwoven material. These types of laminates are often used in order to achieve a soft and a textile-like surface of the backing sheet. In order to accomplish an airier and comfortable product it is also possible to use breathable backing sheets, which prevents fluid from coming out of the absorbent product, but that allows moisture to be ventilated. These breathable backing sheets may be composed of single material layers, or of laminates of, for example, blown or moulded polyethylene films, which have been laminated with, for example, a nonwoven layer of spunbond or of spunbond-meltblown-spunbond (SMS).

The absorption body is typically built up by one or more layers of cellulose fibres, for example cellulose fluff pulp. Other materials, which may be used, are for example absorbing nonwoven material, foam material, synthetic fibre materials or peat. In addition to cellulose fibres or other absorbing materials, the absorbent body may also comprise superabsorbent material, so called SAP (super absorbent polymers), that is material in the form of fibres, particles, granula, film or the like, which material has the ability to absorb fluid corresponding to several times the weight of the superabsorbent material. The superabsorbent material binds the fluid and forms a fluid-containing gel. Moreover, the absorbent body may comprise binders, form-stabilising components or the like. Additional layers improving the properties (such as the absorbent properties by spreading liquid in x, y and z direction for a better utilization of the absorbent core) may also be used, such as various types of fluid-spreading material layers or inserts, so called waddings. The absorbent body may be chemically or physically treated in order to change the absorption properties. For instance, it is possible to provide an absorbent layer with compressed regions and/or being compressed in the entire layer(s) in order to control the fluid flow in the absorbent body. It is also possible to enclose the absorbent layer(s) in an envelope of for example tissue material.

Typically, the absorbent body has in its longitudinal direction an outstretched form, and may for example be essentially rectangular, T-shaped or hourglass-shaped. An hourglass-shaped absorbent body is wider in the front and rear parts than in the crotch part, in order to provide an efficient fluid absorption simultaneously as the design facilitates the product to form and to close around the user, thereby giving a better fit around the legs.

In order to further prevent fluid or faeces to leak out, the absorbent product on the side that is facing the wearer may also be equipped with inner fluid barriers, which are attached in connection to the longitudinal edges inside the outer barriers. Preferably, the inner barriers are made of an essentially liquid-impermeable material, such as for example a hydrophobic nonwoven or a plastic film, and are formed as a longitudinal path with a first edge being connected to the absorbent product and a second free edge, which is adapted for being in close contact with the user at use of the absorbent product. The second edge is equipped with one or more elastic elements, preferably an elastic thread, which in contracted state contracts the free edge, whereby an upstanding barrier is formed. The inner barrier may be designed as a strip of a single sheet, wherein the free edge is turned down in order to enclose the elastic element to prevent direct contact of the elastic thread to the user. Alternatively, the barrier may be formed of two combined layers, whereby the elastic thread is attached to the edge of the free end between the two layers. In this case, the inner layer of the barrier may be composed of an elongation of the top sheet and the outer layer of an essentially liquid-impermeable material, or the inner and outer layers of the barrier may be composed of one single material strip, which is folded around the elastic thread.

The rear and/or front parts of the product may also be equipped with a so called waist elastics, which is composed of elastic organs applied along the front and/or rear end edges in order to give the product a soft and flexible enclosure around the waist of the user. Suitably, the elastic organs are attached between the backing sheet and the top sheet with glue or through welding, such as ultra-sonic welding. The elastic organs may be composed of one or more elastic threads, which in a stretched state are applied between the sheets, and thereby form the waist elastics. Alternatively, the elastics may be applied between the sheets in an unstretched state, whereby both sheets instead are gathered or wrinkled at application. Another typical variant of the elastics, which is suitable, is elastic foam material composed of a thin strip of for example polyurethane foam, which like the elastic threads can be applied between the two sheets. Of course, it is also possible to position the elastic organs for the waist elastics on the outside of the backing sheet or on the inside of the top sheet.

Optionally, the absorbent product of the present invention is equipped with barrier flaps (also called "standing gathers"). The main purpose of the barrier flaps is to prevent leakage of fluid from the absorbent product. Therefore, it is important that they provide a good fit to the wearer of the diaper. The barrier flaps have a proximal edge, which is close to the absorbent body and a free distal edge, which contacts the body of the user to provide the fluid barrier and also includes the elastic means. Preferably, the barrier flaps extend along the entire length of the absorbent core, but that may in some cases not be necessary, as long as they provide a secure prevention against leakage. The height of the barrier flaps is preferably 10-50 mm, and both the proximal edge and the distal edge could be joined to the top-sheet in the front and rear ends of the product.

The barrier flaps are kept upstanding by the elastic means, which preferably runs along the distal edge inside the fold of the top sheet, which forms the flaps. This elastic means may be of any kind that is conventional in the art, and which fits into the flap.

The wings or flaps are the parts of some absorbent products that are formed by the backing sheet material, the top sheet material, a combination thereof, or another material, and that extends from the longitudinal sides of the product. The purpose of the wings is to allow attachment of the product to the underpants of the wearer. Generally, the flaps (or wings) extend laterally from a central absorbent means and are intended to be folded around the wearer's panties in the crotch region.

The beard is the parts of the backing sheet and top sheet material that extends outside the longitudinal edges of the absorbent core and are sealed together at the longitudinal sides of some absorbent products. For example, the pantyliners of figure 1 are equipped with a "beard".

The belt is the part of some products (e.g. diapers and incontinence protections for adults) that runs along the waist of the wearer in order to attach the rear and front portions of the product. See e.g. EP-A-1137384 for a more detailed description.

The absorbent product may comprise a fastening system. This fastening system may be of any kind, which is suitable for the product, such as a hook and loop system, or a tape.

In yet another embodiment, the absorbent core is equipped with a wicking layer, which wicking layer has the purpose to spread fluid towards the front part of the absorbent structure. Moreover, the wicking layer does not necessarily need to cover the whole absorbent core, but should preferably cover at least the part of the absorbent core being in the front part of the casing, more preferably the part being in the front and crotch parts of the casing, and most preferably the entire absorbent core.

The wicking layer is of a moisture permeable material, preferably tissue paper or a hydrophilic non-woven, and functions to disperse the fluid, i. e. urine, passing through the liquid permeable top sheet, preferably in a direction towards the front part of the diaper. The wicking layer comprises small capillaries directing the fluid towards smaller capillaries, due to capillary forces.

## Claims

1. Absorbent product, such as a diaper, a sanitary napkin or an incontinence product, comprising a back sheet, being distal from the body of the wearer in use of the product, and a top sheet, being proximal to the body of the wearer in use of the product, the product further comprising an absorbent structure, between the top and back sheet, whereby the product optionally comprises standing gathers, belt, elastics and/or wings, and whereby the product comprises odour control agent(s), whereby the product has a wet zone, which is intended to store liquid during use, which zone is defined by the absorbent structure, a dry zone, which is intended to be substantially dry during use, which zone is defined by the outer edges, belt, elastics, backing sheet, wings and optionally outer part of the standing gathers, and an interfacing zone adjoining the wet zone, which is defined by the part(s) of the top sheet receiving liquid, optionally the inner part of the standing gathers, optionally an acquisition layer, and other parts adjoining the wet zone thereby occasionally being wet, with at least one substantially moisture-insensitive odour control agent present in the wet zone and at least one odour control agent present in the dry zone, wherein the odour control agent of the dry zone is applied in positions where the air of the product is pressed out of the product during use, **characterized in that** said positions are defined by the outer edges, the belt, the fastening means, the elastics, the wings, the outer parts of the standing gathers, and/or the side panels, and wherein at least one odour control agent of the wet zone is directed to bacterial inhibition.

2. Absorbent product according to claim 1, wherein the odour control agents of the wet zone and/or the interfacing zone are (1) acidic SAP in combination with esters and/or chitosan or (2) a modified starch-based odour control agent, chosen from linear dextrin, activated, amylose such as V-amylose and cyclodextrin.

3. Absorbent product according to claims 1 or 2 wherein the odour control agent(s) of the dry zone is(are) chosen from a modified starch based-odour control agent, such as activated starch, V-amylose, especially a modified starch-based odour control agent having a specific area higher than 5 m²/g and/or hydrophobic compounds.

4. Absorbent product according to any one of the preceding claims, wherein the odour control agent(s) of the interfacing zone is/are chosen from acidic pulp and V-amylose.

5. Absorbent product according to claim 4, wherein the odour control agent(s) of the dry zone is/are applied (a) as strips, (b) by being incorporated in or on the fibres of at least a part of the dry zone, (c) as one or more spots, (d) by being coated to the surface of the material, or a combination thereof.

6. Absorbent product according to any one of the preceding claims, wherein the wet zone comprises the odour control agents acidic SAP and at least one of esters and chitosan, preferably cyclic esters or esters chosen from isomenthyl acetate, isomenthyl propionate, isomenthyl isobutyrate, isomenthyl crotonate, isomenthyl butyrate, the dry zone comprises modified starches and/or hydrophobic compounds, and the interfacing zone comprises acidic pulp and/or modified starches, such as V-amylose.

7. Absorbent product according to any one of the preceding claims, whereby at least one groove comprising a foam-based material is formed in the absorbent structure in order to enable transport of odours to a desired location of the dry zone.

8. Absorbent product according to claim 7, whereby the groove has a lateral breadth in the interval from 5-20 mm.

## Patentansprüche

1. Absorptionsprodukt, wie zum Beispiel eine Windel, eine Binde oder ein Inkontinenzprodukt, umfassend eine Rückenschicht distal von dem Körper des Trägers bei Verwendung des Produkts und eine Oberschicht proximal zu dem Körper des Trägers bei Verwendung des Produkts, wobei das Produkt ferner eine Absorptionsstruktur zwischen der Ober- und Rückenschicht umfasst, wobei das Produkt optional stehende Krausen, ein Band, Elastiken und/oder Flügel umfasst und wobei das Produkt Geruchssteuerungswirkstoff(e) umfasst, wobei das Produkt eine feuchte Zone, die dazu gedacht ist, bei Verwendung eine Flüssigkeit zu speichern, welche Zone durch die Absorptionsstruktur definiert ist, eine trockene Zone, die dazu gedacht ist, bei Verwendung im Wesentlichen trocken zu sein, welche Zone durch die äußeren Kanten, das Band, die Elastiken, Rückenschicht, Flügel und optional den äußeren Teil der stehenden Krausen definiert ist, und eine Zwischenzone aufweist, die an die feuchte Zone anliegt, welche durch den Teil (die Teile) der Oberschicht, der Flüssigkeit aufnimmt (aufnehmen), optional den inneren Teil der stehenden Krausen, optional eine Erfassungsschicht und andere Teile, die an der feuchten Zone anliegen, die dadurch gelegentlich feucht sind, definiert ist, mit zumindest einem im Wesentlichen feuchtigkeitsunsensiblen Geruchssteuerungswirkstoff, der in der feuchten Zone vorliegt, und zumindest einem Geruchssteuerungswirkstoff, der in der trockenen Zone vorliegt, wobei der Geruchsteuerungswirkstoff der trockenen Zone an Positionen aufgebracht wird, wo die Luft des Produkts bei Verwendung aus dem Produkt herausgedrückt wird, **dadurch gekennzeichnet, dass** diese Positionen durch die äußeren Kanten, das Band, die Befestigungsmittel, die Elastiken, die Flügel, die äußeren Teile der stehenden Krausen und/oder die Seitenfelder definiert sind, und wobei mindestens ein Geruchssteuerungswirkstoff der feuchten Zone zur Bakterienhemmung ausgerichtet ist.

2. Absorptionsprodukt nach Anspruch 1, wobei die Geruchssteuerungswirkstoffe der feuchten Zone und/oder der Zwischenzone (1) saures SAP in Verbindung mit Estern und/oder Chitosan oder (2) ein modifizierter stärkebasierter Geruchssteuerungswirkstoff, ausgewählt aus linearem Dextrin, aktivierter Amylose, wie zum Beispiel V-Amylose, und Cyclodextrin sind.

3. Absorptionsprodukt nach Anspruch 1 oder 2, wobei der (die) Geruchssteuerungswirkstoffe(e) der trockenen Zone aus einem modifizierten stärkebasierten Geruchssteuerungswirkstoff, wie zum Beispiel aktivierter Stärke, V-Amylose, insbesondere einem modifizierten stärkebasierten Geruchssteuerungswirkstoff mit einer höheren spezifischen Fläche als 5m²/g und/oder hydrophoben Verbindungen, ausgewählt ist (sind).

4. Absorptionsprodukt nach einem der vorhergehenden Ansprüche, wobei der (die) Geruchssteuerungswirkstoff(e) der Zwischenzone aus einer sauren Pulpe und V-Amylose ausgewählt ist/sind.

5. Absorptionsprodukt nach Anspruch 4, wobei der (die) Geruchssteuerungswirkstoff(e) der trockenen Zone (a) als Streifen, (b) durch Einarbeiten in oder auf die Fasern zumindest eines Teils der trockenen Zone, (c) als ein oder mehrere Punkte, (d) durch Beschichten auf die Oberfläche des Materials oder eine Kombination hiervon aufgetragen wird/werden.

6. Absorptionsprodukt nach einem der vorhergehenden Ansprüche, wobei die feuchte Zone die Geruchssteuerungswirkstoffe saures SAP und zumindest ein von Ester oder Chitosan, bevorzugt zyklische Ester oder Ester, aufweist, die aus Isomenthylacetat, Isomenthylpropionat, Isomenthylisobutyrat, Isomenthylcrotonat, Isomenthylbutyrat ausgewählt sind, die trockene Zone modifizierte Stärken und/oder hydrophobe Verbindungen aufweist und die Zwischenzone saure Pulpe und/oder modifizierte Stärken, wie zum Beispiel V-Amylose, aufweist.

7. Absorptionsprodukt nach einem der vorhergehenden Ansprüche, wobei zumindest eine Nut, umfassend ein schaumbasiertes Material, in der Absorptionsstruktur ausgebildet ist, um einen Transport von Gerüchen zu einer gewünschten Stelle der Trockenzone zu ermöglichen.

8. Absorptionsprodukt nach Anspruch 7, wobei die Nut eine seitliche Breite im Intervall von 5-20 mm aufweist.

## Revendications

1. Produit absorbant tel qu'une couche, une serviette hygiénique ou un produit pour incontinents, comprenant une feuille d'envers, distale par rapport au corps du porteur lors de l'utilisation du produit, et une feuille supérieure, proximale par rapport au corps du porteur lors de l'utilisation du produit, le produit comprenant en outre une structure absorbante, entre les feuilles supérieure et d'envers, le produit comprenant éventuellement des plis de maintien, une ceinture, des élastiques et/ou des ailettes, et le produit comprenant un ou plusieurs agents de maîtrise des odeurs, le produit ayant une zone humide qui est destinée à stocker le liquide durant l'utilisation, laquelle zone est définie par la structure absorbante, une zone sèche qui est destinée à être pratiquement sèche durant l'utilisation, laquelle zone est définie par les bords extérieurs, la ceinture, les élastiques, la feuille d'envers, les ailettes et éventuellement une partie extérieure des plis de maintien, et une zone d'interface attenante à la zone humide, qui est définie par la ou les parties de la feuille supérieure recevant le liquide, éventuellement la partie intérieure des plis de maintien, éventuellement une couche d'acquisition, et d'autres parties attenantes à la zone humide qui sont donc occasionnellement humides, au moins un agent de maîtrise des odeurs pratiquement insensible à l'humidité étant présent dans la zone humide, et au moins un agent de maîtrise des odeurs étant présent dans la zone sèche, l'agent de maîtrise des odeurs dans la zone sèche étant appliqué en des positions où l'air du produit est expulsé du produit durant l'utilisation, **caractérisé en ce que** lesdites positions sont définies par les bords extérieurs, la ceinture, les moyens de fixation, les élastiques, les ailettes, les parties extérieures des plis de maintien, et/ou les panneaux latéraux, et au moins un agent de maîtrise des odeurs de la zone humide étant ciblé vers une inhibition bactérienne.

2. Produit absorbant selon la revendication 1, dans lequel les agents de maîtrise des odeurs de la zone humide et/ou de la zone d'interface sont (1) un SAP acide en combinaison avec des esters et/ou du chitosane ou (2) un agent de maîtrise des odeurs à base d'amidon modifié, choisi parmi la dextrine linéaire, une amylose activée telle que la V-amylose et la cyclodextrine.

3. Produit absorbant selon les revendications 1 ou 2, dans lequel le ou les agents de maîtrise des odeurs de la zone sèche est/sont choisis parmi un agent de maîtrise des odeurs à base d'amidon modifié, tel que l'amidon activé, la V-amylose, en particulier un agent de maîtrise des odeurs à base d'amidon modifié ayant une surface spécifique supérieure à 5 m²/g et/ou des composés hydrophobes.

4. Produit absorbant selon l'une quelconque des revendications précédentes, dans lequel le ou les agents de maîtrise des odeurs de la zone d'interface est/sont choisis parmi la pâte acide et la V-amylose.

5. Produit absorbant selon la revendication 4, dans lequel le ou les agents de maîtrise des odeurs de la zone sèche est/sont appliqué(s) (a) sous la forme de bandes, (b) en étant incorporés dans ou sur les fibres d'au moins une partie de la zone sèche, (c) sous la forme d'un ou plusieurs points, (d) en étant déposés sous forme de revêtement sur la surface du matériau, ou une de leurs combinaisons.

6. Produit absorbant selon l'une quelconque des revendications précédentes, dans lequel la zone humide comprend les agents de maîtrise des odeurs de type SAP acide et au moins l'un parmi des esters et le chitosane, de préférence des esters cycliques ou des esters choisis parmi l'acétate d'isomenthyle, le propionate d'isomenthyle, l'isobutyrate d'isomenthyle, le crotonate d'isomenthyle, le butyrate d'isomenthyle, la zone sèche comprend des amidons modifiés et/ou des composés hydrophobes, et la zone d'interface comprend de la pâte acide et/ou des amidons modifiés, tels que la V-amylose.

7. Produit absorbant selon l'une quelconque des revendications précédentes, dans lequel au moins une rainure comprenant un matériau à base de mousse est formée dans la structure absorbante afin de permettre le transport d'odeurs vers un emplacement souhaité de la zone sèche.

8. Produit absorbant selon la revendication 7, dans lequel la rainure a une largeur latérale située dans l'intervalle allant de 5 à 20 mm.
